(19) Europäisches Patentamt
European Patent Office
Office européen des brevets

(11) Publication number: **0 279 477 B1**

(12) **EUROPEAN PATENT SPECIFICATION**

(45) Date of publication of patent specification: **06.05.92**

(51) Int. Cl.[5]: **C07C 67/38**, C07C 69/24

(21) Application number: **88200128.2**

(22) Date of filing: **26.01.88**

(54) **Carbonylation process.**

(30) Priority: **29.01.87 GB 8701966**

(43) Date of publication of application:
**24.08.88 Bulletin 88/34**

(45) Publication of the grant of the patent:
**06.05.92 Bulletin 92/19**

(84) Designated Contracting States:
**BE DE ES FR GB IT NL**

(56) References cited:
**EP-A- 0 043 382**
**EP-A- 0 055 875**
**EP-A- 0 186 228**
**US-A- 4 414 409**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Petrus, Leonardus**
**Badhuisweg 3**
**NL-1031 CM Amsterdam(NL)**

EP 0 279 477 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a continuous process for the carbonylation of an alkene or an alkyne.

Carbonylation processes are well known processes in the art, e.g. it is known that ethylene, carbon monoxide and methanol react to obtain methyl propionate.

Representative carbonylation processes are disclosed in EP-A-186,228; US-A-4,414,409; EP-A-43382; and EP-A-55876. These processes are run batch-wise.

A number of catalyst systems are at present known which promote the carbonylation reaction. It is known that Group VIII noble metals (e.g. palladium) enhance the reaction. In order to have a homogeneous system a palladium compound is used in combination with a ligand and an acid. In a continuous carbonylation process the catalyst system tends to decline in activity. The loss of activity of the catalyst is caused by the formation of catalytically inactive compounds, formed by ligands and acids with the starting materials, such as ethylene or methanol.

The present invention relates to a continuous process for the carbonylation of an alkene or an alkyne which comprises adding an alkene or an alkyne, carbon monoxide and a hydroxyl group containing compound to a reactor, reacting the starting materials in the presence of a catalytic system comprising palladium or a palladium compound, a ligand selected from organic phosphines, and an acid, whilst monitoring the product concentration, and adding to the reactor from $10^{-6}$ to $10^{-2}$ millimole of both ligand and acid per mole of product, either continuously or intermittently.

Suitable alkenes are alkenes having from 2 to 20 carbon atoms, such as ethene, propene, 1-butene, 2-butene, isobutene, the isomeric pentenes, hexenes, octenes, dodecenes, cyclododecene, 1,5-cyclooctadiene, 1,5,9-cyclododecatriene and styrene. Suitable alkynes comprise acetylene or mono- or di-alkyl substituted acetylene, e.g. propyne.

For the process of the present invention alkenes are preferably used and more preferably ethene or propene.

Suitable hydroxylgroup containing compounds comprise water, alcohols and carboxylic acids which may be aliphatic, cycloaliphatic or aromatic. The alcohol may also be a phenol. The alcohols or carboxylic acids preferably contain not more than 20 carbon atoms. Examples of suitable alcohols are methanol, ethanol, propanol, isobutanol, tert. butanol, stearyl alcohol, benzyl alcohol, cyclohexanol, allyl alcohol, ethylene glycol, 1,2-propanediol, 1,4-butanediol, glycerol, polyethylene glycol, 1,6-hexanediol, phenol and cresol. Preference is given to alcohols having from 1 to 6 carbon atoms. If the alcohol has more than one hydroxyl group, different products may be formed, depending on the molar ratios existing between the reagents. For instance, depending on the quantity of alkene, mono-esters or diesters may be produced predominantly from glycerol.

Other suitable hydroxyl group containing compounds are hydroxy sulphonates of the general formula (I) $HO\text{-}R\text{-}SO_3M$ wherein R represents a divalent optionally substituted hydrocarbyl group and M represents a metal of Group I or II of the Periodic Table of the Elements.

The hydroxy sulphonates of the general formula (I) $HO\text{-}R\text{-}SO_3M$ to be used in the process of the present invention are those in which R represents a divalent hydrocarbyl group containing preferably not more than 20 carbon atoms. The hydrocarbyl group may be aliphatic, cycloaliphatic or aromatic and may be substituted with one or more substituents. The symbol M of the general formula (I) may represent a metal of Group I or II of the Periodic Table of the Elements such as Na, K, Cs, Ca, Mg, Cu or Zn. Preferably M represents the alkali metals Na or K.

Examples of suitable hydroxy sulphonates are sodium or potassium salts of 2-hydroxyethanesulphonic acid, 3-hydroxypropanesulphonic acid, 3-hydroxybutanesulphonic acid, 4-hydroxybenzenesulphonic acid, 2-hydroxybenzenesulphonic acid, 4-hydroxy-3-methylbenzenesulphonic acid, 4-hydroxy-2,6-dimethylbenzenesulphonic acid, 5-hydroxy-3-methyl-2-propylbensenesulphonic acid, 4-hydroxy-3-methoxybenzenesulphonic acid, 5-hydroxy-4-methoxy-2-methylbenzenesulphonic acid, 2-hydroxy-6-nonylbenzenesulphonic acid and 2,4-dihydroxybenzenesulphonic acid. The use of hydroxy sulphonates of the general formula (I) wherein R represents a phenylene group is preferred.

The palladium catalysts suitable to be used in the process according to the present invention are based on palladium salts of for instance, nitric acid, sulphuric acid or alkane carboxylic acids having not more than 12 carbon atoms. Salts of hydrohalogenic acids may be used as well. Preference is given to palladium(II)-acetate. Moreover, palladium complexes may be used, for instance palladium acetylacetonate, tetrakistriphenylphosphinepalladium, bis-tri-o-tolylphosphinepalladium acetate or bis-triphenylphosphinepalladium sulphate.

The quantity of the palladium catalyst may vary within wide ranges. Preference is given to the use of quantities in the range between $10^{-5}$ and $10^{-1}$ gram atom palladium per mol of alkene or alkyne.

The ligand generally contains one phosphorus atom. Special attention is drawn to organic phosphines. Very suitable phosphines are those of the general formula (II) $PR^1R^2R^3$ in which $R^1$, $R^2$ and $R^3$ are substituted or unsubstituted phenyl groups. Suitable substituents are halogen atoms and alkyl, aryl, alkoxy, carboxy, carbalkoxy, acyl, trihalogenmethyl, cyano, dialkylamino, sulphonylalkyl and alkanoyloxy groups.

Examples of suitable phosphines are tri-p-tolylphosphine, tri-p-methoxyphosphine, o-diphenylphosphinobenzoic acid, and in particular triphenylphosphine. The phosphine is preferably used in a molar excess of the quantity of palladium, more preferably at least 5 mol, still more preferably at least 10 mol per gram atom of palladium. If the palladium catalyst already contains phosphine, this should be taken into account when calculating the amount of phosphine to be used.

The acids to be used as promoters in the process according to the present invention preferably have a non-coordinating anion, by which is meant that little or no co-valent interaction takes place between palladium and the appropriate anion. Typical examples of such anions are $PF_6^-$, $SbF_6^-$, $BF_4^-$ and $ClO_4^-$.

Acids preferably used are, for instance, sulphonic acids and those acids that can be formed, possibly in situ, by interaction of a Lewis acid such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulphonic, phosphoric acid or sulphuric acid. Specific examples of the last-named type of acids are fluorosilicic acid, $HBF_4$, $HPF_6$ and $HSbF_6$. Typical sulphonic acids that can be used are fluorosulphonic acid, chlorosulphonic acid and the sulphonic acids specified hereinafter.

A preferred group of acids has the general formula III

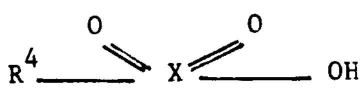

(III)

wherein X represents sulphur or chlorine and, if X is chlorine, $R^4$ represents oxygen and if X is sulphur, $R^4$ represents an OH group or an optionally substituted hydrocarbyl group.

When the afore-mentioned acids are used in the process according to the invention, the anions of the acids can be regarded as non-coordinating.

In the acids having the general formula III, the optionally substituted hydrocarbyl group, represented by $R^4$, is preferably an alkyl, aryl, aralkyl or alkaryl group with 1-30, in particular 1-14, carbon atoms. The hydrocarbyl group may be substituted with for instance halogen atoms, in particular fluorine atoms, hydroxy groups or acyl groups. Examples of suitable acids of the general formula III are perchloric acid, sulphuric acid, 2-hydroxypropane-2-sulphonic acid, p-toluenesulphonic acid and trifluoromethane sulphonic acid.

The quantity of acid used in the carbonylation reaction may vary within ranges. Generally the amount of acid is 1 to 50 mol of acid per gram atom of palladium.

Generally the amount of ligand applied is in excess of the amount of acid, calculated on a molar basis, although for carrying out the reaction this is not necessary.

To maintain a constant activity of the catalyst in a continuous process ligand and acid are preferably continuously supplied. Generally the amount of acid and ligand, necessary to maintain constant activity ranges from $10^{-6}$ to $10^{-2}$ mol of each per mol of product.

The ratio of the molar amounts of ligand and acid during the reaction period ranges from 0.75 to 3.0. At the begin of the reaction this molar ratio will normally be in the range of from 1 to 3 and during the reaction the molar ratio of the added ligand and acid will be in the range of from 0.75 to 1.25 and is more preferably about 1.

The process according to the present invention can be carried out using an aromatic hydrocarbon as reaction medium. Examples of suitable aromatic hydrocarbons are benzene, toluene, o-, m- or p-xylene, ethylbenzene, pentylbenzene, mesitylene, chlorobenzene, cumene, anisole and diphenyl ethers. Preference is given to the ester product as a solvent.

Generally the obtained reaction mixture is (continuously) conducted to a separation unit e.g. a distillation unit and the carbonylated product is distilled overhead and the remaining bottom product, containing the catalyst components is recycled to the reactor.

In order to avoid the presence or built up of a large concentration of catalytically inactive compounds, derived from catalyst ingredients, it is preferred to remove a part of the bottom product from the system. It may be removed from the bottom of the distillation column. The other part which is led back into the reactor is generally admixed with the hydroxyl group containing compound, e.g. methanol.

In the process according to the present invention carbon monoxide may be used pure or diluted with an inert gas, such as nitrogen, noble gases or carbon dioxide. The presence of small amounts of hydrogen is generally not detrimental to the carbonylation reaction.

The carbonylation process is preferably carried out at a temperature in the range between 50 ° and 200 ° C, in particular between 80 ° and 160 ° C. The overall pressure preferably lies between 15 and 75 bar.

EXAMPLE

A continuous process for the preparation of methyl propionate was carried out in a system comprising a 250 ml autoclave equipped with a magnetically driven stirrer, a degaser and a distillation unit. The autoclave was provided with inlets for carbon monoxide, ethylene, fresh and recycled catalyst and with an outlet. Liquid and excess of gas could leave the autoclave via a dip tube at the liquid level, passing through a back-pressure regulator and a capillary flow restriction towards an atmospheric degaser. The liquid leaving this degaser was passed to a distillation unit operating under atmospheric pressure. The catalyst containing bottom stream leaving this distillation unit was recycled to the autoclave. As top products of the distillation methanol and methyl propionate were collected. All gases leaving the degaser and distillation unit passed through a cold trap and a recording wet gas meter. The gas consumption rate was used as a measure of the catalytic activity. Initially the autoclave was charged with 0.25 mmol palladium acetate, 15 mmol triphenylphosphine and 5 mmol methanesulphonic acid. The initial reaction conditions were a temperature of 111 ° C and a pressure of 20 bar. In the autoclave was constantly present about 150 ml liquid comprising methyl propionate. The total liquid present in the reactor, atmospheric degaser and distillation unit was 200 ml. The amounts of feed methanol/CO/ethene were 0.59/0.875/0.875 mol/h respectively. The recycle stream from the distillation unit to the reactor was 2 ml/min. The rate of addition of triphenylphosphine and methanesulphonic acid each was 0.2 mmol/h. This addition was necessary since catalytically inactive phosphonium salts were found.

The methanol conversion during 166 hours was constant at about 40%. Byproduct formation was extremely low, the selectivity to methylpropionate being 99.8%. The main byproduct is 4-oxohexanoic acid methyl ester. At run hour 166 the addition of triphenylphosphine and methanesulphonic acid was terminated, and as a result thereof a sharp decline of catalyst activity was observed.

**Claims**

**1.** A continuous process for the carbonylation of an alkene or an alkyne, which comprises adding an alkene or an alkyne, carbon monoxide and a hydroxyl group containing compound to a reactor, reacting the starting materials in the presence of a catalytic system comprising palladium or a palladium compound, a ligand selected from organic phosphines, and an acid, whilst monitoring the product concentration, and adding to the reactor from $10^{-6}$ to $10^{-2}$ millimole of both ligand and acid per mole of product, either continuously or intermittently.

**2.** A process according to claim 1 wherein an alkene is used as starting material.

**3.** A process according to claim 1 wherein ethene, acetylene, propene or propyne is carbonylated.

**4.** A process according to any one of the preceding claims wherein the hydroxyl group containing compound is an alcohol having one to six carbon atoms per molecule.

**5.** A process according to any one of the preceding claims wherein the ligand is a triarylphosphine.

**6.** A process according to any one of the preceding claims wherein the ratio of the molar amounts of added ligand and added acid ranges from 0.75 to 1.25.

**7.** A process according to claim 6 wherein equimolar amounts of ligand and acid are added during the reaction.

**8.** A process according to any one of the preceding claims wherein the reaction mixture is conducted to a distillation unit and wherein the carbonylated product is distilled overhead and part of all of the remaining bottom product containing catalyst components, is recycled to the reactor.

**Revendications**

1. Un procédé continu pour la carbonylation d'un alcène ou d'un alcyne, selon lequel on introduit un alcène ou un alcyne, de l'oxyde de carbone et un composé hydroxylé dans un réacteur, on fait réagir les matières de départ en présence d'un système catalytique comprenant du palladium ou un composé du palladium, un ligand choisi parmi des phosphines organiques et un acide, tout en contrôlant la concentration du produit, et on ajoute dans le réacteur de $10^{-6}$ à $10^{-2}$ millimole tant de ligant que d'acide par mole de produit, de manière continue ou intermittente.

2. Un procédé selon la revendication 1, dans lequel on utilise un alcène comme matière de départ.

3. Un procédé selon la revendication 1, dans lequel on carbonyle de l'éthène, de l'acétylène, du propène ou du propyne.

4. Un procédé selon l'une quelconque des revendications précédentes, dans lequel le composé hydroxylé est un alcool ayant un à six atomes de carbone par molécule.

5. Un procédé selon l'une quelconque des revendications précédentes, dans lequel le ligand est une triarylphosphine.

6. Un procédé selon l'une quelconque des revendications précédentes, dans lequel le rapport des quantités molaires de ligand ajouté et d'acide ajouté est compris entre 0,75 et 1,25.

7. Un procédé selon la revendication 6, dans lequel des quantités équimolaires de ligand et d'acide sont ajoutées durant la réaction.

8. Un procédé selon l'une quelconque des revendications précédentes, dans lequel le mélange de réaction est conduit à une unité de distillation et le produit carbonylé est distillé en tête et on recycle au réacteur une partie ou la totalité du résidu de distillation contenant les constituants du catalyseur.

**Patentansprüche**

1. Kontinuierliches Verfahren zur Carbonylierung eines Alkens oder eines Alkins, umfassend die Zugabe eines Alkens oder eines Alkins, von Kohlenmonoxid und einer hydroxylgruppenhaltigen Verbindung in einen Reaktor, Umsetzung der Ausgangsmaterialien in Gegenwart eines katalytischen Systems, umfassend Palladium oder eine Palladiumverbindung, einen Liganden ausgewählt aus organischen Phosphinen, und eine Säure, wobei die Produkt-Konzentration überwacht wird, und Einbringen von $10^{-6}$ bis $10^{-2}$ mmol sowohl von dem Liganden als auch der Säure pro Mol Produkt, entweder kontinuierlich oder intermittierend in den Reaktor.

2. Verfahren nach Anspruch 1, wobei ein Alken als Ausgangsmaterial verwendet wird.

3. Verfahren nach Anspruch 1, wobei Ethen, Acetylen, Propen oder Propin carbonyliert wird.

4. Verfahren nach einem der vorangehenden Ansprüche, wobei die hydroxylgruppenhaltige Verbindung ein Alkohol mit 1 bis 6 Kohlenstoffatomen im Molekül ist.

5. Verfahren nach einem der vorangehenden Ansprüche, wobei der Ligand ein Triarylphosphin ist.

6. Verfahren nach einem der vorangehenden Ansprüche, wobei das Verhältnis der molaren Mengen von zugesetztem Liganden und zugesetzter Säure im Bereich von 0,75 bis 1,25 liegt.

7. Verfahren nach Anspruch 6, wobei äquimolare Mengen von Ligand und Säure während der Reaktion zugesetzt werden.

**8.** Verfahren nach einem der vorangehenden Ansprüche, wobei das Reaktionsgemisch in eine Destillationseinheit geführt wird und das carbonylierte Produkt über Kopf abgezogen wird und ein Teil oder das gesamte verbleibende Bodenprodukt, das die Katalysatorkomponenten enthält, wieder in den Reaktor zurückgeführt wird.